# EUROPEAN PATENT SPECIFICATION

(11) **EP 1 582 593 B1**
(45) Date of publication and mention of the grant of the patent: **15.04.2009**
(21) Application number: 04020552.8
(22) Date of filing: 30.08.2004
(51) Int. Cl.: C12N 15/89, C12M 3/00

(54) **Apparatus and chip for injecting substance into particle**
Vorrichtung und Chip zur Injektion von Substanzen in Partikel
Appareil et puce pour l'injection d'une substance dans une particule

(30) Priority: 29.03.2004 JP 2004097068
(43) Date of publication of application: 05.10.2005
(73) Proprietor: FUJITSU LIMITED, Kawasaki-shi, Kanagawa 211-8588 (JP)
(72) Inventor: Sasaki, Jun, Nakahara-ku, Kawasaki-shi, Kanagawa 211-8588 (JP); Youoku, Sachihiro, Nakahara-ku, Kawasaki-shi, Kanagawa 211-8588 (JP); Tamamushi, Kazuo, Nakahara-ku, Kawasaki-shi, Kanagawa 211-8588 (JP); Ito, Akio, Nakahara-ku, Kawasaki-shi, Kanagawa 211-8588 (JP)
(74) Representative: HOFFMANN EITLE

(56) References cited:
- EP-A- 1 479 759
- WO-A-00/20554
- US-B1- 6 653 136

## Description

### BACKGROUND OF THE INVENTION

### 1) Field of the Invention

The present invention relates to an apparatus and a chip for injecting a substance into a cell-like particle. The substance may, for example, be a gene solution or a drug solution and the object is, for example, a cell or a cell-like micro particle.

### 2) Description of the Related Art

Recently, in the field of life science, specifically in the fields of the regenerative medicine and the genome-based drug discovery, by injecting a gene solution and a drug solution (hereinafter, "substance") into a cell and a cell-like micro particle (hereinafter, "particle"), modification of property of a particle is practiced. Such a technology enables to elucidate a function of a gene, and also enables a tailor-made medicine for providing a care suitable for a genetic characteristic of an each person.

Conventionally, various technologies for the substance injection have been proposed. Specifically, there are a biological method such as a virus vector method, a chemical method such as a lypofection method, an electric method such as an electroporation method, a physical method such as a particle gun method, an optical method such as a laser injection method, and a mechanical method such as an injection method.

To apply the substance injection technology in the medical field, it is required that the technology does not limit the type of the particle and the substance, and that realizes high injection efficiency and mass production of the injection processed particles. Further, in the medical field, it is also important that the technology is applicable to various forms of injections, such as injection of the substance selected from among a large number of candidates, and injection of more than one kind of the substances into the particle.

For the substance injection technology that can satisfy such the requirements, the injection method is most suitable. The microinjection method is a method in which a drug solution that is filled in a micro needle (with a tip having an external diameter of 1 micrometer (µm) and an internal diameter of 0.5 µm) is injected into a particle (diameter is 10 µm to 100 µm) by injecting the needle in the particle (refer, for example, to the patent literature 1 or the patent literature 2). In this method, the needle tip can be controlled to minimize damage to the particle by carrying out the injection under a microscope with a skilled operator, and a control device with high resolution. Therefore, nearly 100% success rate can be obtained. This method also has an advantage in which this method does not limit a combination of the particle and the substance.

However, there is a disadvantage even in this injection method. That is, throughput is low. In the injection method, even a skilled operator can work on maximum several hundreds of the particles per hour. Concretely, because it is a manual work that is carried out while observing the particles on a petri dish through a microscope besides requiring a skill, even the skilled operator can handle only several hundreds of the particles per hour. Moreover, it also requires the operator himself/herself to carry the petri dish to a cultivation apparatus to forward to a cultivation process. This also lowers the throughput.

To make up this drawback, some proposals have been made. For example, a method in which a plurality of the particles are arranged in a one-dimensional or two-dimensional array, and a plurality of the micro needles that are also arranged in an array in the same way as the particles are arranged, are simultaneously inserted in the particles (refer, for example, to the patent literature 3) has been proposed.

Moreover, a method in which the particle is trapped in a trap that is arranged in one-dimensional array on a side of a silicon base, and the substance is injected in the particles while moving a capillary within the depth of focus, and while observing from above (refer, for example, to the patent literature 4) has also been proposed.

Patent Literature 1: Japanese Patent Application Laid-Open No. 1993-192171.

Patent Literature 2: Japanese Patent Application Laid-Open No. 1994-343478.

Patent Literature 3: Japanese Patent Application Laid-Open No. 2000-23657.

Patent Literature 4: Japanese Patent Application Laid-Open No. 2002-27969.

However, in the method in which the particles and the minute needles are arranged in an array, there have been cases in which the minute needle is not properly inserted in the particle, or in which the particle is damaged by the minute needle because the shape of the particles are not strictly uniform, and variation in relationship in relative positions of each of the particles and each of the micro needles arranged in the array is caused.

In the method in which the substance is injected into the particle trapped while observing from above, it is difficult to improve the throughput sufficiently because there is an extra work in which the operator has to set and remove the particle.

Furthermore, either of the methods described above is intended to take a single form of the substance injection in which a same kind of the substance is injected into a same kind of the particles. Therefore, it is difficult to apply those methods to the substance injection in various forms, such as a form in which more than one kind of the substances are injected into the particle.

WO-A-0020554 discloses a combination of features falling within the broadest possible meaning of the preamble of claim 1.

EP-A-1 479 759 is an intermediate patent document in the present technical field.

### SUMMARY OF THE INVENTION

According to the present invention, there is provided a chip for use in an apparatus for injecting a substance into a cell-like particle by injecting a needle into a particle, comprising: a base; a charging unit that is formed in the base and in which the particle is to be charged; an extracting unit that is formed in the base and from which the particle is to be extracted; a flow path that is formed in the base and is configured and arranged to be used for transporting the particle from the charging unit to the extracting unit; and a transporting unit that is configured and arranged to transport the particle from the charging unit to the extracting unit, characterised in that: the flow path has a movable side-wall that is configured to operate as the transporting unit; the chip further comprises a side-wall moving unit that is configured to move the side-wall to transport the particle in the flow path; and the flow path is arranged so that injection of the substance by the needle is carried out while the particle is in the flow path.

A chip according to the present invention may be used in an apparatus for injecting a substance into a particle by injecting a needle into the particle.

### BRIEF DESCRIPTION OF THE DRAWINGS

To enable a better understanding of the present invention, and to show how the same may be carried into effects reference will now be made, by way of example only, to the accompanying drawings, in which:- .
Fig. 1 is a diagram for explaining a configuration of an apparatus for injecting a substance into a particle and a chip for substance injection according to a first embodiment that explains and does not form part of the invention;
Fig. 2 is a cross-section taken along a line A-A in Fig. 1;
Fig. 3 is a cross-section taken along a line B-B in Fig. 1;
Fig. 4 is a cross-section taken along a line C-C in Fig. 1;
Fig. 5 is a diagram for explaining a configuration of an apparatus for injecting a substance into a particle and a chip for substance injection according to a second embodiment that explains and does not form part of the invention;
Fig. 6 is a diagram for explaining a configuration of an apparatus for injecting a substance into a particle and a chip for substance injection according to a third embodiment that explains and does not form part of the invention;
Fig. 7 is a diagram for explaining a condition when a different branch flow path from a case in Fig. 6 is selected;
Fig. 8 is a cross-section of a transporting unit according to a fourth embodiment that forms part of the claimed invention;
Fig. 9 is a cross-section for explaining a sequential action of the transporting unit of Fig. 8;
Fig. 10 is a cross-section for explaining the sequential action of the transporting unit of Fig. 8;
Fig. 11 is a cross-section of a substance injecting unit according to fifth embodiment not forming part of the invention, but useful for understanding a modified embodiment of the invention near a needle;
Fig. 12 is a cross-section of a substance injecting unit according to sixth embodiment not forming part of the invention, but useful for understanding a modified embodiment of the invention near a needle;
Fig. 13 is a cross-section of the substance injecting unit in Fig. 12 in a state in which the needle is injected in the particle;
Fig. 14 is a cross-section of a substance injecting unit according to seventh embodiment not forming part of the invention, but useful for understanding a modified embodiment of the invention; and
Fig. 15 is a cross-section of the substance injecting unit in Fig. 14 in a state in which a needle is injected in the particle.

### DETAILED DESCRIPTION

Exemplary embodiments of an apparatus and a chip are explained below with reference to the accompanying drawings.

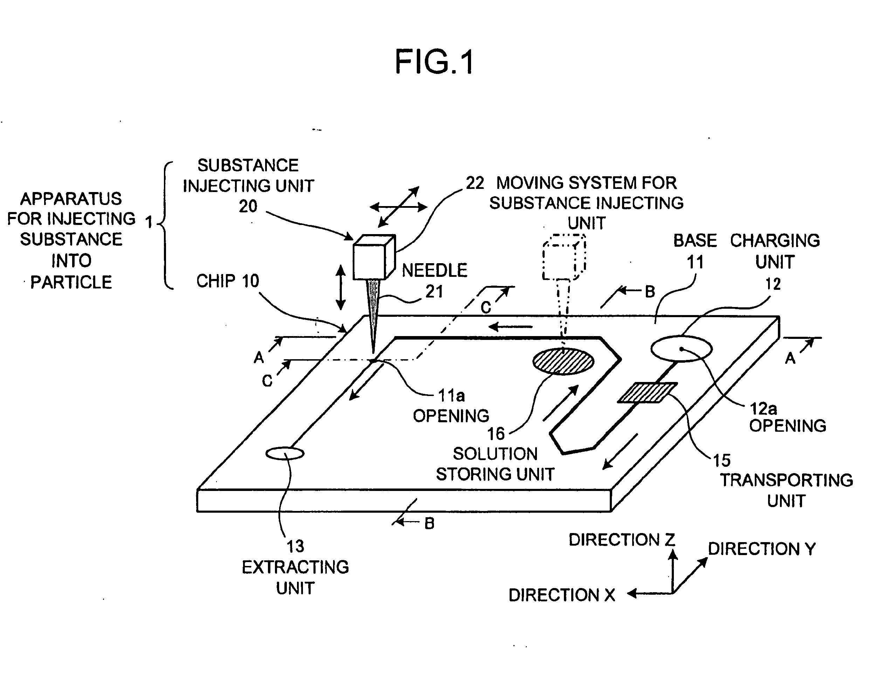

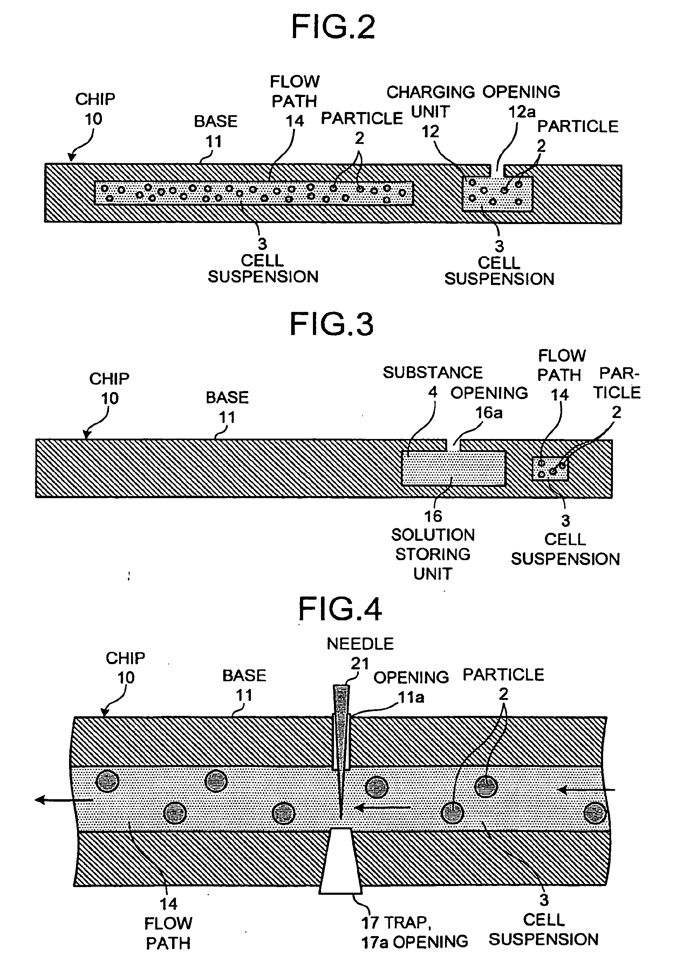

An apparatus and a chip for injecting a substance into a particle according to a first embodiment useful for explaining the invention are explained first. Fig. 1 is a diagram for explaining a configuration of the apparatus and the chip. Fig. 2 is a cross-section taken along a line A-A shown in Fig. 1. Fig. 3 is a cross-section taken along a line B-B shown in Fig. 1. Fig. 4 is a cross-section taken along a line C-C shown in Fig. 1. As shown in Fig. 1, an apparatus 1 includes a chip 10 and a substance injecting unit 20.

The chip 10 includes a base 11, a charging unit 12, an extracting unit 13, a flow path 14, a transporting unit 15, and a solution storing unit 16. An XYZ coordinate system shown Fig. 1 is used. In other words, two directions that cross at right angles on a side parallel to the base 11 (or a base 81 described later) are X and Y directions, and a direction that cross at right angle to both the direction X and the direction Y is the Z direction.

The base 11 is the basic structure of the chip 10. The charging ' unit 12, the extracting unit 13, the flow path 14, the transporting unit 15, and the solution storing unit 16 are formed on and within the base 11. Although some of these components are not exposed on the surface of the base 11, each of these components is shown in a solid line in Fig. 1 and Figs. 5 to 7 for simplicity of explanation.

The base 11 can be of any form. For example the base 11 may be flat and square-shaped as shown in Fig. 1. The base 11 can be of any size. For example, the base 11 is of a size of a glass slide for a microscope. Moreover, the base 11 can be formed with any material. For example, the base 11 may be formed with glass and transparent resin such as polycarbonate and silicone rubber. It is preferable that material of the base 11 is transparent so that it is easy to observe the condition inside or on the surface of the base 11 with a microscope and an image pickup device such as a camera.

Any known method can be used to form the charging unit 12, the extracting unit 13, the flow path 14, the transporting unit 15, and the solution storing unit 16 in the base 11. For example, each of these components may be formed by laser processing the base 11. Each of these components and the base 11 may also be formed into one piece by molding. Molding is preferable because it is simpler.

The charging unit 12 is a place where a cell suspension 3 that contains particles 2 (see Fig. 2) is charged (i.e., placed or accommodated). As shown in Fig. 2, the charging unit 12 is a hollow in the base 11 and has an opening 12a. The cell suspension 3 is injected, for example, with a syringe, in the charging unit 12 through the opening 12a. The charging unit 12 can have any form. For example, the charging unit 12 may be cylindrical. The position of the charging unit 12 is arbitrary. However, it is preferable that the charging unit 12 is near a corner of the base 11, as shown in Fig. 1, which allows an effective use of the space in the base 11.

The extracting unit 13 is a unit to extract the particle 2 from the base 11. The extracting unit 13 is a hollow in the base 11. The cell suspension 3 that is transported through the flow path 14 is introduced in the extracting unit 13. The cell suspension 3 introduced into the extracting unit 13 can be extracted, for example, with a syringe, through an opening (not shown). The position of the extracting unit 13 is arbitrary. However, it is preferable that the extracting unit 13 is near a corner of the base 11, as shown in Fig. 1, which allows an effective use of the space in the base 11.

The flow path 14 is a transporting path through which the particle 2 in the cell suspension 3 is transported from the charging unit 12 to the extracting unit 13. The flow path 14 is like a hollow pipe inside the base 11 as shown in Figs. 2 and 3, and it connects the charging unit 12 to the extracting unit 13. The flow path 14 guides, to the extracting unit 13, the cell suspension 3 that is injected into the charging unit 12, and that is transported by the transporting unit 15.

The flow path 14 can be of any shape. It may have a square cross-section as shown in Figs. 2 and 3 or it may have a circular cross-section. Although a specific course of the flow path 14 is also arbitrary, it is preferable that the flow path 14 is formed with straight and curved portions to improve space efficiency in the base 11. However, to transport the cell suspension 3 smoothly, it is preferable that a curvature of the curved portions is not too small.

Moreover, although a specific width and depth of the flow path 14 is arbitrary as long as the cell suspension 3 can be transported there-through, it is preferable that the flow path 14 is formed to have such a width and depth that only one particle 2, which is included in the cell suspension, is passable at a time because it makes it easier to trap the particle 2 by a trap 17 described later. For example, if the cell suspension 3 that includes the particle 2 of which the diameter is of the order of 15 micrometer (µm) is used, it is preferable that the flow path is formed in the width of 30 µm to 100 µm and in the depth of 30 µm to 50 µm.

The transporting unit 15 is a unit to transport the particle 2 from the charging unit 12 to the extracting unit 13. A specific structure of the transporting unit 15 is arbitrary, and in the first embodiment, µm transporting unit 15 is structured with a syringe pump. The cell suspension 3 is compression transported to the extracting unit 13 by positive pressure or negative pressure applied to the cell suspension 3 that passes through the flow path 14. Although a position of the transporting unit 15 is arbitrary, if the positive pressure is applied with the syringe pump by the transporting unit 15, by arranging the transporting unit 15 at a position that is up-stream in the flow path 14 to a position at which the substance injecting unit 20 is arranged (a position between the charging unit 12 and the substance injecting unit 20), it becomes possible to transport the cell suspension 3 at least to the position of the substance injecting unit 20. On the other hand, if the negative pressure is to be applied by the transporting unit 15, by arranging the transporting unit 15 at a position that is down-stream in the flow path 14 to the position at which the substance injecting unit 20 is arranged (a position between the substance injecting unit 20 and the extracting unit 13), it becomes possible to transport the cell suspension 3 at least to the position of the substance injecting unit 20.

The solution storing unit 16 is for storing a substance 4 in such a manner that the substance 4 can be extracted when necessary. The solution storing unit 16 is a hollow in the base 11 as shown in Fig. 3. The substance 4 can be injected, for example, with a syringe, in the solution storing unit 16 through the opening 16a. The substance injecting unit 20 sucks the substance 4 from the solution storing unit 16 through the opening 16a, and then injects it into the particle 2.

The solution storing unit 16 may have any form. For example, the solution storing unit 16 may be substantially cylindrical. The position of the solution storing unit 16 is arbitrary. However, it is preferable that the position of the solution storing unit 16 is such that the substance 4 can be efficiently transported to the substance injecting unit 20. For example, if the substance injecting unit 20 is movable in the direction X from the position shown in the figure, it is preferable that the solution storing unit 16 is arranged next to the substance injecting unit 20 in the direction X.

An amount of a substance 4 that can be injected into the solution storing unit 16 can be determined considering the work efficiency. For example, the amount is determined so that the substance 4 that can be filled at one time in the solution storing unit 16 enables an operator to carry out a predetermined number of the substance injection on a predetermined number of the particles 2.

The trap 17 is formed in the chip. The trap 17 is used when trapping the particle 2 in the flow path 14. As shown in Fig. 4, an opening 17a is formed at the bottom of the base 11. The opening 17a has a diameter that is smaller than a minimum diameter of the particle 2. For example, if the diameter of the particle 2 is about 15 µm, it is preferable that the diameter of the opening 17a is between of 4 µm and 5 µm. A suction device (not shown) is connected to the opening 17a and sucks the cell suspension 3 that flows in the flow path 14. As a result, the particle 2 in the cell suspension 3 is trapped at the opening 17a. The suction device is, for example, a syringe pump.

The substance injecting unit 20 is used to inject the substance 4 into the particle 2. The substance injecting unit 20 includes a needle 21 and a moving system 22 that moves the substance injecting unit 20 in the XYZ coordinate system.

The needle 21 may be any known needle used for such purpose. For example, for the particle 2 having a diameter from 10 µm to 100 µm, the needle 21 that may be used may have tip with an external diameter of 1 µm and an internal diameter of 0.5 µm. As shown in Fig. 4, an opening 11a is formed on an upper side of the base 11, and through this opening 11a, the tip of the needle 21 is inserted in the flow path 14. Although a diameter of the opening 11a can be arbitrarily determined as long as the tip of the needle 21 can pass through, it is preferable that the diameter is about 20 µm.

As shown in Fig. 4, the opening 11a and the opening 17a are on the same plane. Therefore, it is possible to insert the tip of the needle 21 into the particle 2 just by moving the needle 21 in the Z direction.

The moving system 22 moves the needle 21 in the X, Y, and Z directions. Any known actuator can be used as the moving system 22. Specifically, in the first embodiment, the moving system 22 can move the needle 21 in parallel to the base 11, and on a line between the position shown in Fig. 1 (hereinafter, an "injecting position") and a position shown in a phantom line (a position reached by moving in parallel to the direction X from the position shown in Fig. 1, and that is above the solution storing unit 16, hereinafter, a "sucking position") in the same figure. Therefore, after the substance 4 stored in the solution storing unit 16 is sucked into the needle 21 at the sucking position, the needle 21 is moved to the injecting position, and the substance 4 can be injected into the particle 2 by injecting the needle 21 at the injecting position.

How the substance is injected in the particle is explained next. First, an operator injects the cell suspension 3 into the charging unit 12 through the opening 12a. The operator also injects the substance 4 into the solution storing unit 16 through the opening 16a. When the operator starts the transporting unit 15 with a predetermined method, the cell suspension 3 is introduced toward the extracting unit 13 through the flow path 14 by transporting force of the transporting unit 15. On the other hand, the needle 21 is shifted to the sucking position by the moving system 22, and the substance 4 stored in the solution storing unit 16 is sucked through the opening 16a into the needle 21. Then, the needle 21 is shifted to the injecting position with the moving system 22.

When the cell suspension 3 passes over the opening 17a, the particle 2 is trapped at the opening 17a due to a suction force by the suction device. In this condition, the needle 21 is moved down, penetrates the particle 2 and the substance 4 in the needle 21 is injected into the particle 2. Such an operation can be automated while observing trapping condition of the particle 2 with an observation device not shown. When the injection is completed, the particle 2 trapped in the trap 17 is released by stopping the suction by the suction device. The released particle 2 flows along with the cell suspension 3 that it is transported to the extracting unit 13. Then, the operator extracts the cell suspension 3 from the extracting unit 13 with a syringe and the like. Thus, the substance injection is completed.

Thus, in the first embodiment, it is possible to automatically carry out the processes from the charging to the extraction of the particle 2. Therefore, the throughput of the substance injection can be drastically improved.

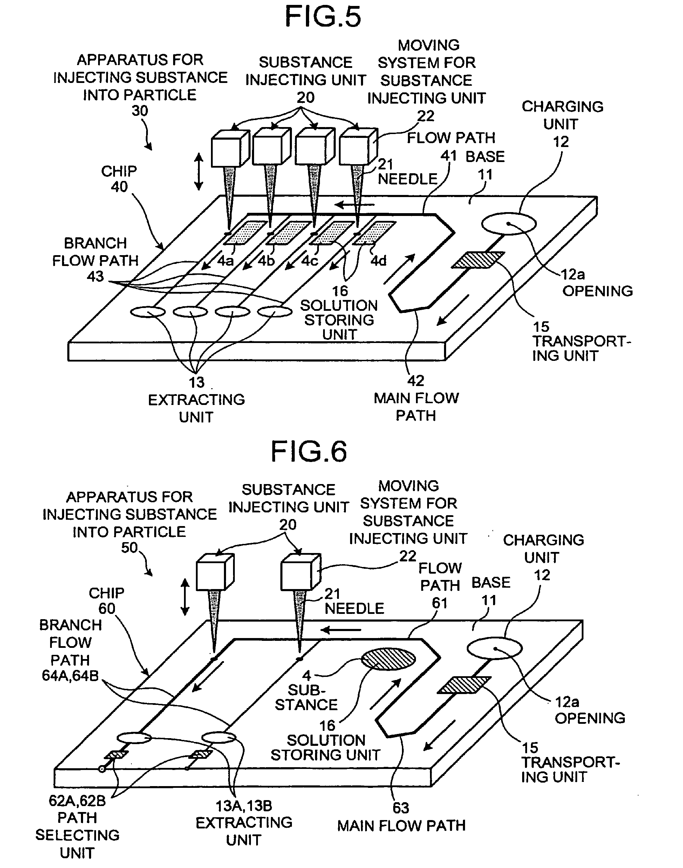

An apparatus and a chip for injecting a substance into a particle according to a second embodiment useful for explaining the invention are explained next. Fig. 5 is a diagram for explaining the apparatus and the chip according to the second embodiment. Structures and methods that are not particularly mentioned here are same as the structures and the methods in the first embodiment. Moreover, like reference characters are given to like components. An apparatus 30 according to the second embodiment includes a chip 40, and a plurality of the substance injecting units 20. The chip 40 includes the base 11 that includes the charging unit 12, a plurality of the extracting units 13, a flow path 41, the transporting unit 15, and a plurality of the solution storing units 16.

The flow path 41 includes a main flow path 42, and a plurality of branch flow paths 43 that correspond to the plurality of the substance injecting units 20. The main flow path 42 is connected to the charging unit 12, and enables the particle 2 (not shown) that is charged in the charging unit 12 to be introduced to the branch flow paths 43. Each of the branch flow paths 43 is connected to the main flow path 42 and the extracting unit 13, and enables the particle 2, which is introduced from the main flow path 42, to be introduced to the extracting unit 13.

The solution storing unit 16 is arranged on a side of each of the branch flow paths 43 in the direction X. In each of the solution storing units 16, a same kind of the substance 4 or each different kind of the substance 4 may be stored as necessary. If the same kind of the substance 4 is stored, it is possible to inject the same kind of the substance 4 into a plurality of the particles 2 at one time. If the different kinds of the substances 4 from each other are stored, it is possible to inject various kinds of the substances into the particles 2 at one time.

Each of the branch flow paths 43 include a trap 17 (not shown). The substance injecting unit 20 is arranged at each of the branch flow paths 43. The substance injecting unit 20 includes the needle 21, and the moving system 22. The substance injecting unit 20 is moved between the injecting position and the sucking position by the moving system 22.

Procedure of the substance injection by the apparatus 30 is explained next. First, the operator injects the cell suspension 3 into the charging unit 12, and injects the same kind or the different kinds of substances 4a to 4d into the solution storing units 16. When the operator starts the transporting unit 15 with the predetermined method, the cell suspension 3 is introduced into the main flow path 42, and into the plurality of the branch flow paths 43 by the transporting force of the transporting unit 15. On the other hand, the needle 21 of each of the substance injecting units 20 is shifted to the sucking position for each by the moving system 22, and each of the substances 4a to 4d stored in the solution storing units 16 is sucked into the needle 21. Then, the needle 21 is moved to the injecting position for each by the moving system 22.

Then, the particle 2 is captured at the trap 17 arranged at each of the branch flow paths 43. In this condition, the needle 21 is shifted down by the moving system 22, and each of the substances 4a to 4d is injected into the particle 2 by injecting the tip of the needle 21 in the particle 2, and by discharging each of the substances 4a to 4d held by the needle 21. When the injection is completed, the particle 2 released from the trap 17 is included in the cell suspension 3 that is to be transported to the extracting unit 13 through each of the branch flow paths 43. Then, the operator extracts the cell suspension 3 from the extracting unit 13 with a syringe and the like that are also not shown. Thus, the substance injection is completed.

Thus, in the second embodiment, it is possible to inject one kind or different kind of the substances 4a to 4d into the particles 2 at one time. Therefore, the substance injection in various forms as required can be carried out.

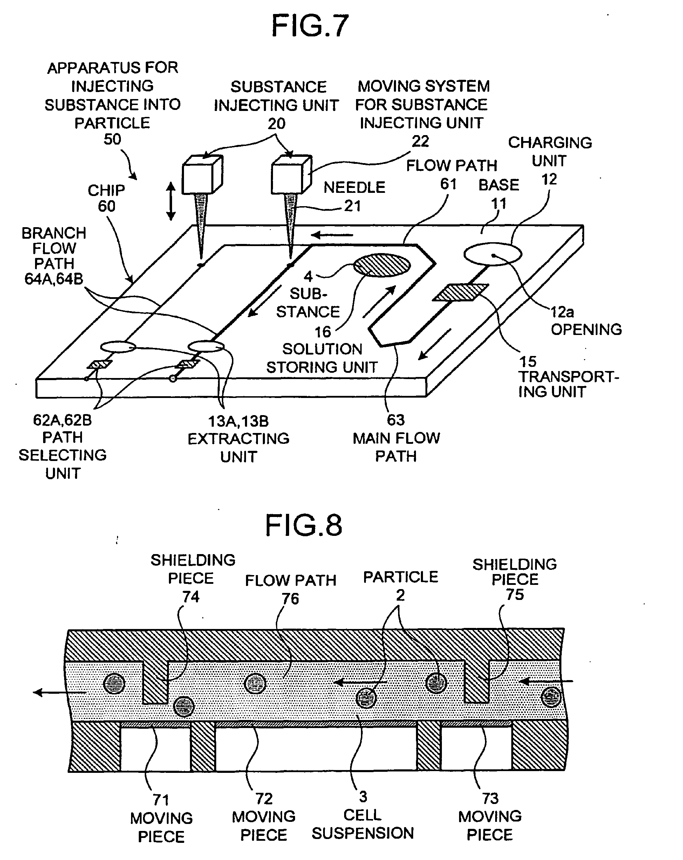

An apparatus and a chip for injecting a substance into a particle according to a third embodiment useful for explaining the invention are explained next. Fig. 6 is a diagram for explaining a configuration of an apparatus and a chip according to the third embodiment, and Fig. 7 is a diagram for explaining a condition when a different branch flow path from a case in Fig. 6 is selected. Structures and methods that are not particularly mentioned are same as the structures and the methods in the second embodiment described above, and like reference characters are given to like components. An apparatus 50 includes a chip 60 and two substance injecting units 20. The chip 60 includes the base 11 that includes the charging unit 12, two extracting units 13A and 13B that correspond to the substance injecting units 20, a flow path 61, the transporting unit 15, the solution storing unit 16, and path selecting units 62A and 62B.

The flow path 61 in the third embodiment includes a main flow path 63 and two paths of branch flow paths 64A and 64B. The substance injecting unit 20 is arranged at the injecting position prepared at each of the branch flow paths 64A and 64B. In the third embodiment, only one of the solution storing unit 16 is arranged, therefore, the same kind of the substance 4 is injected by each of the substance injecting units 20.

The path selecting units 62A and 62B are path selecting units to select the branch flow path 64A or 64B to which the particle 2 is transported from among the branch flow paths 64A and 64B. The path selecting units 62A and 62B may be structured arbitrarily as long as the path selecting units 62A and 62B have a function of selecting one of the branch flow paths 64A and 64B. For example, in the third embodiment, each of the extracting units 13A and 13B is arranged at some midpoint on each of the branch flow paths 64A and 64B, and each of the path selecting units 62A and 62B is arranged on each of the branch flow paths 64A and 64B near the end of the branch flow paths 64A and 64B. The path selecting units 62A and 62B are structured as valves that control transporting of the cell suspension 3 and the particle 2 in the branch flow paths 64A and 64B by closing either of the branch flow paths 64A and 64B.

Procedure of the substance injection by the apparatus 50 is explained next. First, the operator injects the cell suspension 3 into the charging unit 12, and injects the substance 4 in the solution storing units 16. When the operator starts the transporting unit 15 with the predetermined method, the cell suspension 3 is introduced into the main flow path 63 by the transporting force of the transporting unit 15. If the branch flow path 64B is closed by making only the path selecting unit 62B function with a predetermined method, the cell suspension 3 is introduced only into the branch flow path 64A. The needle 21 of the substance injecting unit 20 that is arranged at the sucking position of the branch flow path 64A, in which the cell suspension 3 is introduced, is moved to the sucking position by the moving system 22, and the substance 4 stored in the solution storing unit 16 is sucked into the needle 21. Then, the needle 21 is moved to the injecting position by the moving system 22.

Then, the particle 2 is captured at the trap 17 (not shown) arranged at the branch flow paths 64A. In this condition, the needle 21 is shifted down by the moving system 22, and the substance 4 is injected into the particle 2 by injecting the tip of the needle 21 in the particle 2, and by discharging the substance 4 held by the needle 21. When the injection is completed, the particle 2 released from the trap 17 is included in the cell suspension 3 that is transported to the extracting unit 13A through the branch flow path 64A. Then, the operator extracts the cell suspension 3 from the extracting unit 13A through an opening not shown with a syringe and the like that are also not shown. Thus, the substance injection is completed.

Moreover, if the substance injection is to be carried out with the branch flow path 64B, which is the other one of the branch flow paths, and the substance injecting unit 20, the operator closes the branch flow path 64A by making the path selecting unit 62A, which is the other one of the path selecting units, function with a predetermined method, while opening the path selecting unit 62B, which has been closed. Thus, the cell suspension 3 is introduced only into the branch flow path 64B, and it is possible to carry out the substance injection with the needle 21 of the substance injecting unit 20. The operator extracts the cell suspension3 from the extracting unit 13 through an opening not shown with a syringe and the like also not shown. Thus, the substance injection is completed.

Thus, in the third embodiment, it is possible to carry out the substance injection by selectively switching between the plurality of the branch flow paths 64A and 64B. Therefore, the substance injection in various forms as required can be carried out.

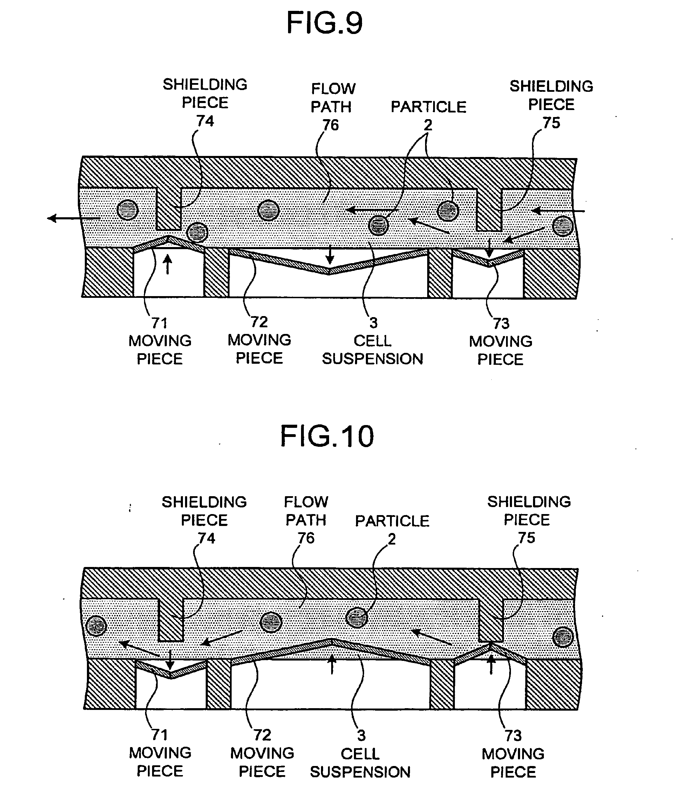

A transporting unit that is used for an apparatus and a chip according to a fourth embodiment, which forms part of the claimed invention, is explained next. Fig. 8 is a cross-section of the transporting unit according to the fourth embodiment. Figs. 9 and 10 are cross-sections of the transporting unit for explaining a sequential action of the transporting unit. Structures and methods that are not particularly mentioned are same as the structures and the methods in the third embodiment described above, and like reference characters are given to like components.

To trap the particle 2 accurately by sucking the cell suspension 3 at the trap as explained previously, it is necessary to retard transporting speed of the particle 2 inside the flow path. To the contrary, to improve the throughput of the substance injection, it is necessary to transport the particle 2 as fast as possible to improve the efficiency of the substance injection. To satisfy these two opposite requirements, it was found in tests that it is preferable that the transporting speed of the particle 2 inside the flow path is 300 µm/s. This speed is equivalent to a speed of about 0.1 µl/min in a flow path that has a width of 100 µm and a depth of 50 µm.

As an example suitable for the transporting unit that can realize such a speed of solution transport, a transporting unit 70, according to the fourth embodiment, that is of a diaphragm pump type is structured. The transporting unit 70 is applicable to the transporting unit 15 of the chip according to the first to third embodiments described above. The transporting unit 70 includes a plurality of moving pieces 71 to 73 and a plurality of shielding pieces 74 and 75 as shown in Fig. 8. The moving pieces 71 to 73 are side wall moving units that can change a volumetric capacity inside a flow path 76 by moving a side wall (shown as a bottom wall here) of the flow path 76. The moving pieces 71 to 73 are formed in thin films, and are aligned toward the direction to which the cell suspension 3 is transported, as shown with arrows in Fig. 8. Each of the moving pieces 71 to 73 is driven to move in the direction (the direction Z) so as to change the volumetric capacity of the flow path 76 by a driving unit not shown.

The shielding pieces 74 and 75 are walls that project from the side wall of the flow path 76 inside the flow path 76 and that at least partially shield flow of the cell suspension 3 inside the flow path 76. The shielding pieces 74 and 75 are arranged at each of portions that correspond to the moving piece 71 arranged in a frontward position and the moving piece 73 arranged in a rearward position on the side wall (shown as an upper wall here) opposite to the side wall at which the moving pieces 71 to 73 are arranged.

Transport of the cell suspension 3 by the transporting unit 70 is carried out as follows. First, the moving piece 71 in the frontward position is moved to project toward inside the flow path 76 as shown in Fig. 9 so that the volumetric capacity of the flow path 76 at the frontward position is reduced. Particularly, the flow path 76 is almost closed at the frontward position with the moving piece 71 and the shielding piece 74. At the same time, the moving pieces 72 and 73 at the central and the rearward positions are moved toward outside the flow path 76 so that the volumetric capacity of the flow path 76 near the central and the rearward positions is enlarged. As a result, the cell suspension 3 that is in up-stream in the flow path 76 to a position of the transporting unit 70 flows into the flow path 76 near the central and the rearward position.

Then, the moving piece 71 at the frontward position is moved toward outside the flow path 76 as shown in Fig. 10 so that the volumetric capacity of the flow path 76 at the frontward position is enlarged. At the same time, the moving pieces 72 and 73 at the central and the rearward positions are moved to project toward inside the flow path 76 so that the volumetric capacity of the flow path 76 at the central and the rearward positions is reduced. Particularly, the flow path 76 is almost closed at the central and the rearward positions with the moving piece 73 and the shielding piece 75. As a result, the cell suspension 3 that has flowed into the flow path 76 at the central and the rear ward positions position is pushed out of the flow path 76 at the central and the rearward positions, and is transported ahead of the transporting unit 70 through the frontward position of the flow path 76. If the cell suspension 3 can be transported without closing the flow path 76 or if the flow path 76 can be closed only with the moving piece 71 and 73 by making the stroke of the moving pieces 71 and 73 larger, the shielding pieces 74 and 75 may be omitted.

Thus, in the fourth embodiment, it is possible to improve the efficiency of the substance injection by transporting the particle 2 as fast as possible while improving the accuracy in trapping the particle 2 by retarding the transporting speed of the particle 2 inside the flow path 76 to some extent. Furthermore, the transporting unit 70 has a compact structure with the moving pieces 71 to 73 that are thin films, therefore, it is possible to arrange in the flow path 76, which is extremely small, in the chip. Further, because flexibility in positioning is high, it is possible to arrange various numbers of the transporting unit 70 at various positions depending on requirements. Therefore, the substance injection in various forms as required can be carried out.

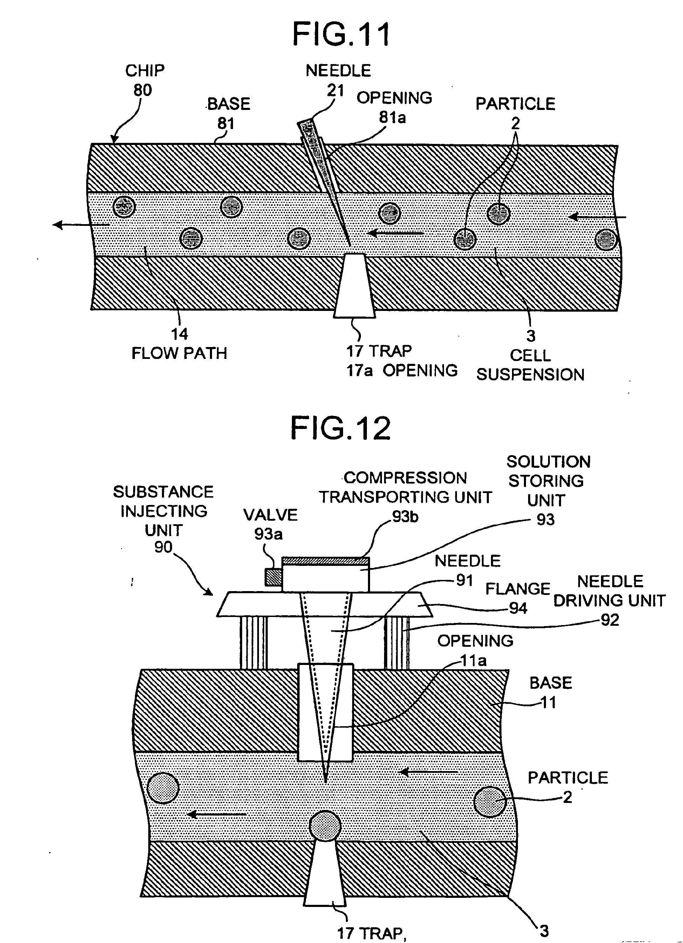

A substance injecting unit used in an apparatus and a chip for injecting a substance into a particle according to a fifth embodiment not forming a part of the claimed invention, but useful for understanding a modified embodiment of the invention is explained next. Fig. 11 is a cross-section of the substance injecting unit according to the fifth embodiment at a portion near the needle. Structures and methods that are not particularly mentioned are same as the structures and the methods in the first embodiment described above, and like reference characters are given to like components.

An opening 81a is formed on an upper surface of a base 81 of an apparatus 80 according to the fifth embodiment. The opening 81a is formed in obliquely (the direction neither parallel nor orthogonal to flowing direction in the path of the flow path) to the flowing direction in the flow path 14 unlike in the first embodiment. The tip of the needle 21 can be inserted obliquely to the base 81 into the flow path through this opening 81a. The opening 17a of the trap is arranged on an extended line in the direction to which the needle 21 is inserted. Therefore, as well as the first embodiment, it is possible to inject the tip of the needle 21 into the particle 2 just by shifting the needle 21 in the direction of the length (in the oblique direction). The substance injecting unit thus structured is applicable to the apparatus and the chip for injecting a substance into a particle according to the first to third embodiments.

Thus, in the fifth embodiment, because the needle 21 is inserted obliquely to the flow path 14, space right above the injecting position becomes open. Therefore, observing the trapping condition of the particle 2 and the like become facilitated.

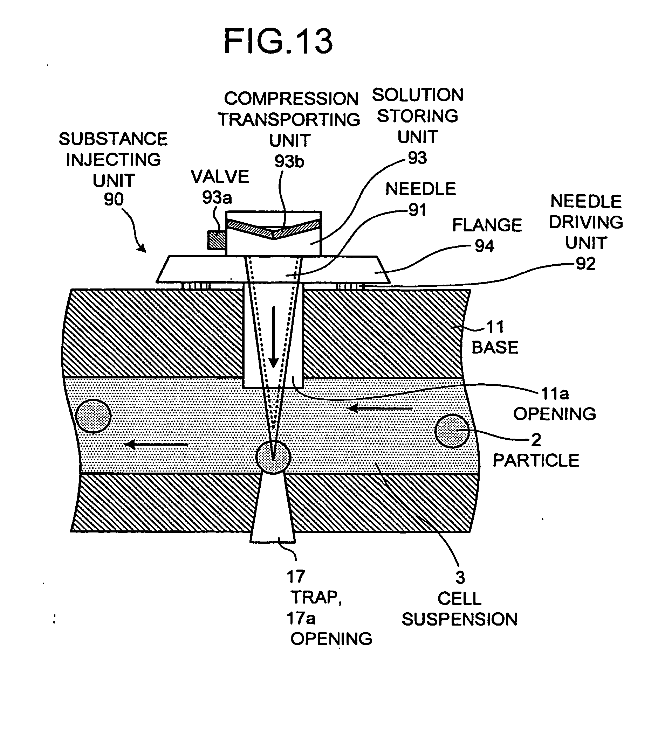

A substance injecting unit used in an apparatus and a chip for injecting a substance into a particle according to a sixth embodiment not forming a part of the claimed invention, but useful for understanding a modified embodiment of the invention is explained next. Fig. 12 is a cross-section near the substance injecting unit according to the sixth embodiment. Fig. 13 is a cross-section of the substance injecting unit shown in Fig. 12 in a state in which the needle is injected in the particle. Structures and methods that are not particularly mentioned are same as the structures and the methods in the first embodiment described above, and like reference characters are given to like components.

A substance injecting unit 90 according to the sixth embodiment includes a needle 91, a needle driving unit 92, and a solution storing unit 93 that are arranged in the base 11. The needle 91 may be structured substantially the same as the needle 21 in the first embodiment, however, in the sixth embodiment in particular, the needle 91 is formed into a hollow cone, and at a thicker side of the cone, a flange 94 is fixed. It is preferable that the needle 91 is arranged, as shown in Fig. 12, at a position on a plane that includes the opening 17a of the trap 17. The needle 91 may also be arranged obliquely to the base as explained in the fifth embodiment.

The needle driving unit 92 is a driving unit that moves the needle 91 along the inserting direction of the needle 91. Specifically, the needle driving unit 92 is a driving unit that supports the needle 91 on the base 11, and of which a form can be changed in a direction along the inserting direction of the needle 91. Concretely, the needle driving unit 92 is formed into a cylinder that reaches from the base 11 to the flange 94. A driving system of the needle driving unit 92 is arbitrary, and, for example, the needle driving unit 92 may be composed of layered piezoelectric devices or bi-metal, and drives the needle 91 in a direction along the inserting direction of the needle 91.

The solution storing unit 93 is a storing unit to store the substance 4 for supplying the substance 4 to the needle 91. The solution storing unit 93 is structured as a hollow micro container, and is fixed to a base of the needle 91 so as to be connected to an internal space of the needle 91. The solution storing unit 93 includes a valve 93a on a side, and through this valve 93a, the substance 4 is injected in the solution storing unit 93 with a syringe and the like not shown. The solution storing unit 93 also includes a compression transporting unit 93b that compression transports the substance 4 stored in the solution storing unit 93 into the needle 91. A compression transport system of the compression transporting unit 93b is arbitrary. For example, a top plate of the solution storing unit 93 may be structured with layered piezoelectric devices or bi-metal as the compression transporting unit 93b, and by causing displacement in the compression transporting unit 93b in the direction toward the needle 91, the compression transporting unit 93b compression transports the substance 4 stored in the solution storing unit 93 into the needle 91.

The substance injection with such the substance injecting unit 90 is carried out as follows. First, the operator injects the substance 4 into the solution storing unit 93 through the valve 93a with a syringe and the like not shown. Then, as shown in Fig. 12, the particle 2 is trapped at the opening 17a of the trap 17. The needle driving unit 92 is started to cause the displacement with a predetermined method, and the needle 91 is moved toward the inserting direction to inject the needle 91 into the particle 2 as shown in Fig. 13. Then, the compression transporting unit 93b is started to cause the displacement with a predetermined method, and the substance 4 stored in the solution storing unit 93 is compression transported into the needle 91. The substance 4 thus compression transported is injected into the particle 2 through the internal space of the needle 91.

Thus, in the sixth embodiment, because the substance injecting unit 90 can be mounted on the base, it is possible to improve flexibility in positioning the substance injecting unit 90, and to make the size of the apparatus compact. Therefore, the substance injection in various forms as required can be carried out.

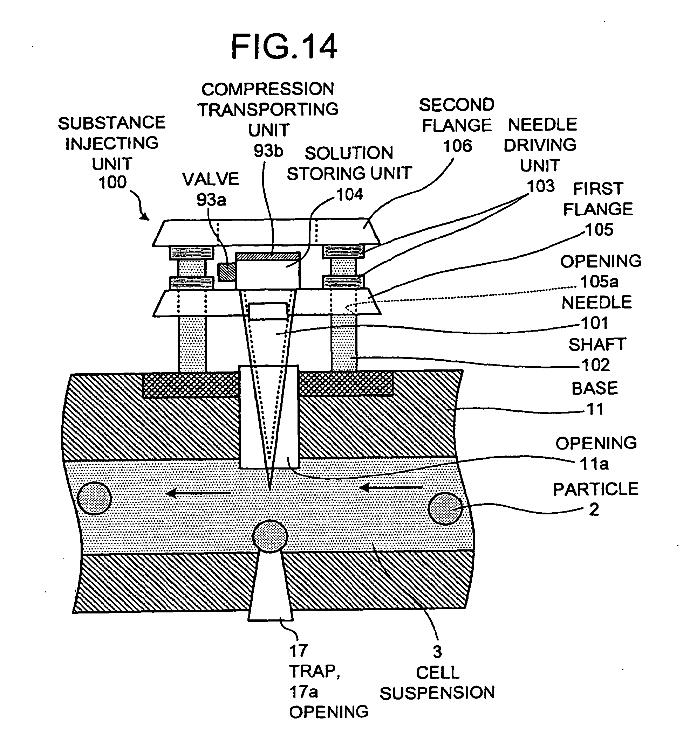

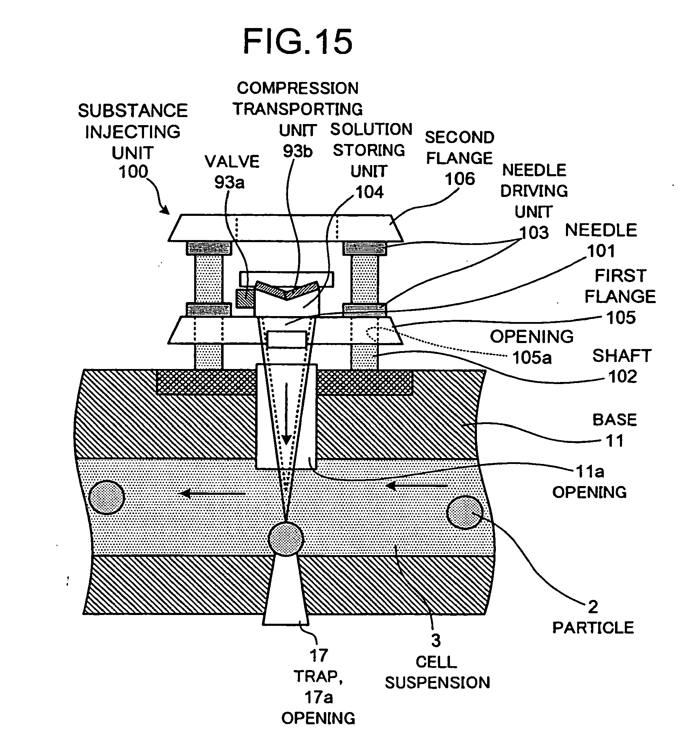

A substance injecting unit used in an apparatus and a chip for injecting a substance into a particle according to a seventh embodiment not forming a part of the claimed invention, but useful for understanding a modified embodiment of the invention is explained next. Fig. 14 is a cross-section near the substance injecting unit according to the seventh embodiment. Fig. 15 is a cross-section of the substance injecting unit shown in Fig. 14 in a state in which the needle is injected in the particle. Structures and methods that are not particularly mentioned are same as the structures and the methods in the sixth embodiment described above, and like reference characters are given to like components.

A substance injecting unit 100 according to the seventh embodiment includes a needle 101, a shaft 102, a needle driving unit 103, and a solution storing unit 104 on the base 11. A first flange 105 is fixed to a base of the needle 101, and an opening 105a that has a diameter that corresponds to a diameter of the shaft is formed in the first flange 105 at a position that corresponds to the shaft 102. The shaft 102 is a supporting unit that movably supports the needle 101 on the base 11. In the seventh embodiment, the shaft 102 is substantially cylindrical, and is arranged on the base 11. The shaft 102 is inserted in the opening 105a of the first flange 105. Thus, the needle 101 can be supported by the shaft 102, and can be moved along the inserting direction of the needle 101. A second flange 106 is fixed to a tip of the shaft 102.

The needle driving unit 103 is arranged between the first flange 105 and the second flange 106, and drives the needle 101 along the inserting direction of the needle 101 by moving the first flange 105 to an opposite direction of the second flange 106. A driving system of the needle driving unit 103 is arbitrary, and for example, the needle driving unit 103 may be structured with a ring-shaped electromagnet or a linier motor to drive the needle 101 by making the first flange 105 electromagnetically repel the second flange 106.

The substance injection with the substance injecting unit 100 is carried out as follows. First, the particle 2 is trapped at the opening 17a of the trap 17 as shown in Fig. 14. Then, the needle driving unit 103 is started to cause the displacement to move the needle 101 along the inserting direction as shown in Fig. 15. Thus, the needle 101 is injected in the particle 2. The compression transporting unit 93b is started to cause the displacement to compression transport the substance 4 stored in the solution storing unit 104 into the needle 101. The substance 4 thus compression transported is injected into the particle 2 through the internal space of the needle 101.

Thus, in the seventh embodiment, because the substance injecting unit 100 can be mounted on the base, it is possible to improve flexibility in positioning the substance injecting unit 100, and to make the size of the apparatus compact. Therefore, the substance injection in various forms as required can be carried out.

All or a part of the control explained as the control that is automatically carried out in each of the embodiments above may be carried out manually. Furthermore, charge of the cell suspension 3 in the charging unit 12 may also be automated with a preceding device connected to the charging unit 12. Moreover, extraction of the cell suspension 3 from the extracting unit 13 may be automated with a succeeding device connected to the extracting unit 13. Charge of the substance 4 in the solution storing unit 16 may also be automated with an automatic injection device connected to the solution storing unit 16.

Furthermore, more than one function may be given to one component. For example, if the charging unit 12 is composed of a syringe pump that can provide the cell suspension 3 into the flow path 14 and the like while keeping a predetermined pressure, the transporting unit 15 may be omitted. Moreover, if some amount of the substance 4 can be stored in the internal space of the needle 21 of the substance injecting unit 20 and the like, the solution storing unit 16 may be omitted.

Furthermore, while in some of the embodiments described above, cases in which the plurality of the branch flow paths 43 are arranged in the flow path 41, and the plurality of the extracting units 13 and the solution storing units 16 that correspond to the branch flow paths 43 has been explained, the number of the charging unit 12 may also be increased to more than one. For example, the charging unit 12 in which only the particle 2 is charged, and the charging unit 12 in which only a cultivation solution is charged may be both prepared, and the particle 2 and the cultivation solution may be supplied to the flow path 41 after mixing the particle 2 and the cultivation solution in a predetermined proportion. Moreover, a plurality of the substance injecting units 20 and the solution storing units 16 may be prepared for one of the flow path 14 and the like to inject the same kind of the substance 4 into just one of the particle 2 providing a time interval, or to inject different kinds of the substances 4 into one of the particle 2. Furthermore, a plurality of the chips 10 may be arranged to form a multilayer chip. This allows efficient use of the space.

With embodiments of the present invention, charge of the particle, injection of the substance, and extraction of the particle are all carried out on one chip. Therefore, it is possible to improve the throughput while employing the injection method that does not limit a type of the particle and the substance.

Furthermore, the substance is contained in the substance containing unit. Therefore, containing the substance can also be carried out on the same chip, and the throughput further improves.

Moreover, the substance that corresponds to each of the branches can be contained in the substance containing unit. Therefore, the substance injection in various forms as required, such as a form in which a different kind of the substance is injected in the particle in each of the branches, can be carried out.

Furthermore, the particle is transferred into the branch that is selected by the path selecting unit. Therefore, the substance injection in various forms as required, such as a form in which a different kind of manipulation is carried out in each of the branches, can be carried out.

Moreover, the charge of the particle, the injection of the substance, and the extraction of the particle are all carried out on one chip. Therefore, it is possible to improve the throughput while employing the injection method that does not limit a type of the particle and the substance.

Furthermore, because a side-wall moving unit that moves the side-wall of the flow path and changes the volumetric capacity of the flow path is provided, it is possible to improve the efficiency of the substance injection by transporting the particle as fast as possible while improving the accuracy in trapping the particle by retarding a transporting speed of the particle inside the flow path to some extent. Moreover, such the unit has a compact structure. Therefore, it is possible to arrange in the flow path, which is extremely small, in the chip. Further, because flexibility in positioning is high, it is possible to arrange the transporting unit at various positions for various numbers depending on requirements. Therefore, the substance injection in various forms as required can be carried out.

Moreover, by providing the trap that traps the particle in the flow path, the needle can be injected in the particle that is trapped. Therefore, it is possible to inject the substance into the particle more accurately, and the throughput improves.

Furthermore, if the opening of the trap is arranged on an extended line in a direction to which the needle is inserted, it is possible to inject the tip of the needle in the particle just by shifting the needle in the direction of the length.

Moreover, if a needle driving unit is provided that supports the needle on the base, and that includes a driving unit that changes height in a direction in which the needle is inserted by changing form , the substance injecting unit can be mounted on the base, and it is possible to improve flexibility in positioning the substance injecting unit, and to make the size of the apparatus compact. Therefore, the substance injection in various forms as required can be carried out.

Furthermore, if the needle is arranged obliquely to the base, a space right above a position in which the needle is injected in the particle becomes open. Therefore, observing the trapping condition of the particle and the like become facilitated.

## Claims

1. A chip for use in an apparatus for injecting a substance into a cell-like particle by injecting a needle into a particle, comprising:
a base (11);
a charging unit (12) that is formed in the base and in which the particle is to be charged;
an extracting unit (13) that is formed in the base and from which the particle is to be extracted;
a flow path (14, 76) that is formed in the base and is configured and arranged to be used for transporting the particle from the charging unit to the extracting unit; and
a transporting unit (15) that is configured and arranged to transport the particle from the charging unit to the extracting unit,
**characterised in that**: the flow path (14, 76) has a movable side-wall (71, 72, 73) that is configured to operate as the transporting unit;
the chip further comprises a side-wall moving unit that is configured to move the side-wall to transport the particle in the flow path; and
the flow path is arranged so that injection of the substance by the needle (21) is carried out while the particle is in the flow path.

2. The chip according to claim 1, further comprising a plurality of branches (43, 64A, 64B) to the flow path.

3. The chip according to claim 1 or 2, wherein the transporting unit (15) is a positive-pressure applying unit that is configured to apply a positive pressure to the particle to transport the particle, and the positive-pressure applying unit is arranged at a position which is up-stream to a position in the flow path (14) at which the injection of the substance by the needle (21) is carried out.

4. The chip according to claim 1, 2 or 3, wherein the transporting unit (15) is a negative-pressure applying unit that is configured to apply a negative pressure to the particle to transport the particle, and the negative-pressure applying unit is arranged at a position which is down-stream to a position in the flow path (14) at which the injection of the substance by the needle (21) is carried out.

5. The chip according to any one of the preceding claims, further comprising a trap (17) that is formed in the flow path and is configured and arranged to trap the particle therein.

6. The chip according to claim 5, wherein the trap (17) includes an opening (17a) that is formed on a wall of the flow path (14), and in which the particle can be trapped by applying a suction force to the opening.

7. The chip according to claim 6, wherein the opening (17a) is arranged at a position on a line that extends from the needle in a direction of movement of the needle.

8. The chip according to any one of the preceding claims, further comprising a substance containing unit (16) that is formed in the base (11) and contains the substance.

9. The chip according to claim 8, further comprising a plurality of branches (43) in the flow path, wherein a substance containing unit (4a, 4b, 4c, 4d) is arranged for each of the branches.

10. The chip according to any one of the preceding claims, further comprising:
a plurality of branches (64A, 64B) to the flow path; and
a path selecting unit (62A, 62B) that is arranged in the base and configured to select a branch to which the particle is transported from among the branches.

11. An apparatus comprising at least a chip according to any preceding claim.

12. The apparatus according to claim 11, wherein the substance injecting unit includes:
a needle driving unit (92, 103) that moves the needle (91, 101) in a desired direction; and
a substance containing unit (16) that is formed in the base and contains the substance.

13. The apparatus according to claim 12, wherein the needle driving unit supports the needle (91, 101) on the base, and includes a driving unit (92, 103) that can change height in the desired direction by changing form.

14. The apparatus according to claim 12, wherein the needle driving unit (92, 103) includes:
a supporting unit (20) that movably supports the needle on the base; and
a driving unit (92, 103) that moves the needle along the supporting unit.

15. The apparatus according to any one of preceding claims 11 to 14, wherein the needle (21) is arranged obliquely to the base.

16. The apparatus according to any one of preceding claims 11 to 15, further comprising a substance containing unit (16) that contains the substance,
wherein the substance injecting unit (20) can be moved in parallel to the base in a space between the substance containing unit (16) and a position at which an injection of the substance into the particle by the needle (21) is carried out.

## Patentansprüche

1. Chip zur Verwendung in einer Vorrichtung zum Einspritzen einer Substanz in ein zellenförmiges Partikel durch Einführen einer Nadel in ein Partikel, umfassend:
eine Basis (11),
eine Ladeeinheit (12), die in der Basis ausgebildet ist und in der das Partikel zu laden ist,
eine Extraktionseinheit (13), die in der Basis ausgebildet ist und aus der das Partikel zu extrahieren ist,
einen Durchflussweg (14, 76), der in der Basis ausgebildet ist und eingerichtet und angeordnet ist, um zum Transportieren des Partikels von der Ladeeinheit zu der Extraktionseinheit verwendet zu werden, und
eine Transporteinheit (15), die eingerichtet und angeordnet ist, um das Partikel von der Ladeeinheit zu der Extraktionseinheit zu transportieren,
**dadurch gekennzeichnet, dass** der Durchflussweg (14, 76) eine bewegliche Seitenwand (71, 72, 73) aufweist, die eingerichtet ist, um als die Transporteinheit zu wirken,
der Chip ferner eine Seitenwand-Bewegungseinheit aufweist, die eingerichtet ist, die Seitenwand zu bewegen, um das Partikel in dem Durchflussweg zu transportieren, und
der Durchflussweg angeordnet ist, damit ein Einspritzen der Substanz durch die Nadel (21) durchgeführt wird, während das Partikel in dem Durchflussweg ist.

2. Chip nach Anspruch 1, ferner umfassend eine Mehrzahl von Zuläufen (43, 64A, 64B) zu dem Durchflussweg.

3. Chip nach Anspruch 1 oder 2, wobei die Transporteinheit (15) eine Überdruckanlegungseinheit ist, die eingerichtet ist, um einen Überdruck auf das Partikel anzulegen, um das Partikel zu transportieren, und die Überdruckanlegungseinheit an einer Position angeordnet ist, die stromaufwärts einer Position in dem Durchflussweg (14) ist, an der das Einspritzen der Substanz durch die Nadel (21) durchgeführt wird.

4. Chip nach Anspruch 1, 2 oder 3, wobei die Transporteinheit (15) eine Unterdruckanlegungseinheit ist, die eingerichtet ist, um einen Unterdruck auf das Partikel anzulegen, um das Partikel zu transportieren, und die Unterdruckanlegungseinheit an einer Position angeordnet ist, die stromabwärts einer Position in dem Durchflussweg (14) ist, an der das Einspritzen der Substanz durch die Nadel (21) durchgeführt wird.

5. Chip nach einem der vorstehenden Ansprüche, ferner umfassend eine Falle (17), die in dem Durchflussweg ausgebildet ist und eingerichtet und angeordnet ist, um das Partikel darin einzufangen.

6. Chip nach Anspruch 5, wobei die Falle (17) eine Öffnung (17a) enthält, die an einer Wand des Durchflusswegs (14) ausgebildet ist und in der das Partikel durch Anlegen einer Saugkraft an die Öffnung eingefangen werden kann.

7. Chip nach Anspruch 6, wobei die Öffnung (17a) an einer Position auf einer Geraden angeordnet ist, die sich von der Nadel in einer Bewegungsrichtung der Nadel erstreckt.

8. Chip nach einem der vorstehenden Ansprüche, ferner umfassend eine Substanzaufnahmeeinheit (16), die in der Basis (11) ausgebildet ist und die Substanz beinhaltet.

9. Chip nach Anspruch 8, ferner umfassend eine Mehrzahl von Zuläufen (43) in dem Durchflussweg, wobei eine Substanzaufnahmeeinheit (4a, 4b, 4c, 4d) für jeden der Zuläufe angeordnet ist.

10. Chip nach einem der vorstehenden Ansprüche, ferner umfassend
eine Mehrzahl von Zuläufen (64A, 64B) des Durchflusswegs und
eine Wegwahleinheit (62A, 62B), die in der Basis angeordnet ist und eingerichtet ist, um einen Zulauf unter den Zuläufen auszuwählen, zu dem das Partikel transportiert wird.

11. Vorrichtung, umfassend zumindest einen Chip nach einem vorstehenden Anspruch.

12. Vorrichtung nach Anspruch 11, wobei die Substanzaufnahmeeinheit
eine Nadelantriebseinheit (92, 103), welche die Nadel (91, 101) in eine gewünschte Richtung bewegt, und
eine Substanz enthaltende Einheit (16), die in der Basis ausgebildet ist und die Substanz beinhaltet, enthält.

13. Vorrichtung nach Anspruch 12, wobei die Nadelantriebseinheit die Nadel (91, 101) auf der Basis hält und eine Antriebseinheit (92, 103) enthält, die eine Höhe in der gewünschten Richtung durch Ändern der Form verändern kann.

14. Vorrichtung nach Anspruch 12, wobei die Nadelantriebseinheit (92, 103)
eine Halteeinheit (20), welche die Nadel beweglich auf der Basis hält, und
eine Antriebseinheit (92, 103), welche die Nadel entlang der Halteeinheit bewegt, enthält.

15. Vorrichtung nach einem der vorstehenden Ansprüche 11 bis 14, wobei die Nadel (21) schräg zu der Basis angeordnet ist.

16. Vorrichtung nach einem der vorstehenden Ansprüche 11 bis 15, ferner umfassend eine Substanzaufnahmeeinheit (16), welche die Substanz beinhaltet,
wobei die Substanz einspritzende Einheit (20) parallel zu der Basis in einem Raum zwischen der Substanzaufbahmeeinheit (16) und der Position bewegt werden kann, bei der ein Einspritzen der Substanz in das Partikel durch die Nadel (21) durchgeführt wird.

## Revendications

1. Puce utilisable dans un appareil pour injecter une substance dans une particule de type cellule en injectant une aiguille dans une particule, comprenant :
une base (11) ;
une unité de chargement (12) qui est formée dans la base et dans laquelle la particule doit être chargée ;
une unité d'extraction (13) qui est formée dans la base et à partir de laquelle la particule doit être extraite ;
un passage d'écoulement (14, 76) qui est formé dans la base et qui est configuré et
disposé de manière à être utilisé pour transporter la particule de l'unité de chargement à l'unité d'extraction ; et
une unité de transport (15) qui est configurée et disposée pour transporter la particule de l'unité de chargement à l'unité d'extraction,
**caractérisée en ce que** :
le passage d'écoulement (14, 76) possède une paroi latérale mobile (71, 72, 73) qui est configurée de manière à fonctionner comme unité de transport ;
la puce comprend en outre une unité de déplacement de paroi latérale qui est configurée pour déplacer la paroi latérale afin de transporter la particule dans le passage d'écoulement ; et
que le passage d'écoulement est disposé de manière à ce que l'injection de la substance avec l'aiguille (21) soit réalisée pendant que la particule est dans le passage d'écoulement.

2. Puce selon la revendication 1, comprenant en outre une pluralité de ramifications (43, 64A, 64B) vers le passage d'écoulement.

3. Puce selon la revendication 1 ou 2, dans laquelle l'unité de transport (15) est une unité appliquant une pression positive qui est configurée pour appliquer une pression positive à la particule pour transporter la particule, et l'unité appliquant une pression positive est disposée à une position qui est en amont d'une position dans le passage d'écoulement (14) où l'injection de la substance avec l'aiguille (21) est réalisée.

4. Puce selon la revendication 1, 2 ou 3, dans laquelle l'unité de transport (15) est une unité appliquant une pression négative qui est configurée pour appliquer une pression négative à la particule pour transporter la particule, et l'unité appliquant une pression négative est disposée à une position qui est en aval d'une position dans le passage pour écoulement (14) où l'injection de la substance par l'aiguille (21) est réalisée.

5. Puce selon l'une quelconque des revendications précédentes, comprenant en outre un piège (17) qui est formé dans le passage d'écoulement et qui est configuré et disposé pour y piéger la particule.

6. Puce selon la revendication 5, dans laquelle le piège (17) comprend une ouverture (17a) qui est formée sur une paroi du passage d'écoulement (14), et dans laquelle la particule peut être piégée en appliquant une forte aspiration à l'ouverture.

7. Puce selon la revendication 6, dans laquelle ouverture (17a) est disposée à une position sur une ligne qui se prolonge à partir de l'aiguille dans une direction de déplacement de l'aiguille.

8. Puce selon l'une quelconque des revendications précédentes, comprenant en outre une unité contenant une substance (16) qui est formée dans la base (11) et qui contient la substance.

9. Puce selon la revendication 8, comprenant en outre une pluralité de ramifications (43) dans le passage d'écoulement, dans laquelle une unité contenant une substance (4a, 4b, 4c, 4d) est disposée dans chacune des ramifications.

10. Puce selon l'une quelconque des revendications précédentes, comprenant en outre :
une pluralité de ramifications (64A, 64B) vers le passage d'écoulement ; et
une unité de sélection de passage (62A, 62B) qui est disposée dans la base et configurée pour sélectionner une ramification vers laquelle la particule est transportée parmi les ramifications.

11. Appareil comprenant au moins une puce selon l'une quelconque des revendications précédentes.

12. Appareil selon la revendication 11, dans lequel l'unité d'injection de substance comprend :
une unité de commande d'aiguille (92, 103) qui déplace l'aiguille (91, 101) dans une direction souhaitée ; et
une unité contenant une substance (16) qui est formée dans la base et qui contient la substance.

13. Appareil selon la revendication 12, dans lequel l'unité de commande d'aiguille supporte l'aiguille (91, 101) sur la base et comprend une unité de commande (92, 103) qui peut changer de hauteur dans la direction souhaitée en changeant de forme.

14. Appareil selon la revendication 12, dans lequel l'unité de commande d'aiguille (92, 103) comprend :
une unité de support (20) qui supporte de manière mobile l'aiguille sur la base ; et
une unité de commande (92, 103) qui déplace l'aiguille le long de l'unité de support.

15. Appareil selon l'une quelconque des revendications 11 à 14 précédentes, dans lequel l'aiguille (21) est disposée à l'oblique par rapport à la base.

16. Appareil selon l'une quelconque des revendications 11 à 15 précédentes, comprenant en outre une unité contenant une substance (16) qui contient la substance, dans lequel l'unité d'injection de substance (20) peut être déplacée parallèlement à la base dans un espace entre l'unité contenant une substance (16) et une position où une l'injection de la substance dans la particule par l'aiguille (21) est réalisée.
